# EUROPEAN PATENT SPECIFICATION

(11) **EP 2 165 609 B1**
(45) Date of publication and mention of the grant of the patent: **04.12.2013**
(21) Application number: 08777110.1
(22) Date of filing: 06.06.2008
(51) Int. Cl.: A23K 1/16, A61P 1/14, A61P 3/00, A61K 36/22

(54) **RUMEN FERMENTATION IMPROVING AGENT**
MITTEL FÜR VERBESSERTE PANSEN-FERMENTIERUNG
AGENT AMÉLIORANT LA FERMENTATION DANS LA PANSE

(30) Priority: 08.06.2007 JP 2007153285; 08.02.2008 JP 2008029494
(43) Date of publication of application: 24.03.2010
(73) Proprietor: Idemitsu Kosan Co., Ltd., Chiyoda-ku Tokyo 100-8321 (JP); National University Corporation Hokkaido University, Sapporo-shi, Hokkaido 060-0808 (JP)
(72) Inventor: KOBAYASHI, Yasuo, Sapporo-shi Hokkaido 060-0809 (JP); NAGASHIMA, Kyo, Sodegaura-shi Chiba 299-0293 (JP); MOCHIZUKI, Masami, Sodegaura-shi Chiba 299-0293 (JP)
(74) Representative: Vossius & Partner
(86) International application number: PCT/JP2008/060490
(87) International publication number: WO 2008/149992

(56) References cited:
- WO-A2-2005/099729
- JP-A- 03 217 484
- JP-A- 08 231 410
- JP-A- 2002 281 912
- JP-A- 2006 166 853
- VAN NEVEL C J ET AL: "EFFECT OF FATTY-ACID DERIVATIVES ON RUMEN METHANE AND PROPIONATE", APPLIED MICROBIOLOGY, vol. 21, no. 2, 1971, pages 365-366, XP002617171, ISSN: 0003-6919
- ISAO KUBO ET AL: "STRUCTURE-ANTIBACTERIAL ACTIVITY RELATIONSHIPS OF ANACARDIC ACIDS", JOURNAL OF AGRICULTURAL AND FOOD CHEMISTRY, AMERICAN CHEMICAL SOCIETY, US, vol. 41, no. 6, 1 June 1993 (1993-06-01), pages 1016-1019, XP000369580, ISSN: 0021-8561, DOI: DOI:10.1021/JF00030A036
- NAGARAJA T G ET AL: "Ruminal Acidosis in Beef Cattle: The Current Microbiological and Nutritional Outlook<1,2>", JOURNAL OF DAIRY SCIENCE, AMERICAN DAIRY SCIENCE ASSOCIATION, US, vol. 90, 1 June 2007 (2007-06-01), pages E17-E38, XP026956032, ISSN: 0022-0302 [retrieved on 2007-06-01]
- SREEMANNARAYANA O ET AL: "A NOTE ON THE PRODUCTION OF VOLATILE FATTY-ACIDS BY BOVINE RUMEN LIQUOR IN ARTIFICIAL RUMEN IN THE PRESENCE OF CASHEW APPLE AND WATER-HYACINTH MEALS", INDIAN JOURNAL OF ANIMAL SCIENCES, vol. 43, no. 6, 1973, XP009143301, ISSN: 0367-8318
- CHENG K J ET AL: "A review of bloat in feedlot cattle", JOURNAL OF ANIMAL SCIENCE, AMERICAN SOCIETY OF ANIMAL SCIENCE, US, vol. 76, no. 1, 1 January 1998 (1998-01-01), pages 299-308, XP008124616, ISSN: 0021-8812
- WATANABE Y ET AL: "In vitro evaluation of cashew nut shell liquid as a methane-inhibiting and propionate-enhancing agent for ruminants", JOURNAL OF DAIRY SCIENCE, vol. 93, no. 11, November 2010 (2010-11), pages 5258-5267, XP009143295, ISSN: 0022-0302
- HIMEJIMA H. ET AL.: 'Antibacterial Agents from the Cashew Anacardium occidentale (Anacardiaceae) Nut Shell Oil' JOURNAL OF AGRICULTURAL AND FOOD CHEMISTRY vol. 39, 1991, pages 418 - 421, XP002419513

## Description

### Technical Field

The present invention relates to the non-therapeutic use of a rumen fermentation improving agent, preferably a feed, containing cashew nut shell liquid.

### Background Art

In ruminants such as cattle and sheep, a feed is digested and fermented by microorganisms in their rumens, and the ruminants live by utilizing the fermentation products. Therefore, it is a loss of energy efficiency when methane is generated from the rumens. Further, because methane is a greenhouse gas that influences the global warming, it is important to reduce the amount of methane produced in the rumens of the ruminants.
A methanogen in a rumen reduces carbon dioxide by utilizing hydrogen to thereby produce methane. The contribution ratio of methane to the global warming is second highest after carbon dioxide, and it is considered that methane emitted from ruminants accounts for 15 to 20% of the total emission of methane.

In 1940s, it was found that the growth of a domestic animal is promoted by adding a small amount of an antibiotic to a domestic animal feed. Since then, the practice of adding the antibiotic to the domestic animal feed has been performed widely as means for promoting the growth of the domestic animal.
It is considered that the antibiotic exhibits an action of promoting the growth by its effects of (1) prevention of pathogenic bacterial infection of a domestic animal, (2) improvement in metabolism, and (3) suppression of proliferation of harmful enterobacteria, but the details thereof are still unclear. On the other hand, as a result of adding antibiotics to a feed, the antibiotics are distributed widely in the environment, and accordingly, an appearance of antibiotic-resistant bacteria has become a social problem. In recent years, the addition of an antibiotic to a feed has been regulated strictly, and in Europe, the use of the antibiotic for promoting the growth was banned by January 2006. Further, there is a strong demand from producers for livestock products that are bred without using an antibiotic, and hence, the need of an alternative to the antibiotic is growing.

Ionophores such as monensin, which are antibiotics, are widely used in a feed for a ruminant. Monensin exhibits a selective suppressing effect with respect to a rumen microorganism and has, as a result, functions of reducing methane production and promoting propionic acid production. Propionic acid has higher ATP production efficiency compared with other volatile fatty acids, and hence, feed efficiency is improved owing to the promotion of propionic acid production.

There is also desired the development of alternatives to monensin and the like to be added to a feed for a ruminant. As the alternatives, there are studied an oil extracted from a plant (Non-patent Document 1), a vaccine against lactic acid-producing bacteria (Non-patent Document 2), an egg yolk antibody against lactic acid-producing bacteria (Non-patent Document 3), and the like. However, those technologies are not in practical use yet, because there are problems in that their effects are not stable, the registration thereof as feeds is not permitted, and the like. Further, there is also studied gluconic acid (Patent Document 1), but the amount of propionic acid production is not exemplified, so a methane production-suppressing effect is not known.

It is known that cashew nut shell liquid has an antibacterial action (Non-patent Document 4) and a coccidiosis-relieving action (Patent Document 2). Further, as for the rumen function-improving effects of a ruminant, an *in vitro* test result using anacardic acid (Non-patent Document 5) is reported, but there is no disclosure on the reproducibility, application, and the optimum content on an actual animal.

Patent Document 1: WO 01/028551
Patent Document 2: JP 08-231410 A
Non-patent Document 1: Benchaar et al., Can. J. Anim. Sci. 86, 91-96 (2006)
Non-patent Document 2: Shu et al., FEMS Immunology & Medical Microbiology, 26(2), 153-158 (1999)
Non-patent Document 3: DiLorenzo et al., J. Anim. Sci., 84, 2178-2185 (2006)
Non-patent Document 4: Muroi, H. et al., Bioorganic & Medicinal Chemistry 12, 583-587 (2004)
Non-patent Document 5: Van Nevel C. J., et al., Applied Microbiology 21, 365-366 (1971)

### Disclosure of the Invention

An object of the present invention is to promote the growth of a ruminant and to improve feed efficiency by improving the rumen fermentation in the ruminant and contributing to suppressing the generation of greenhouse gas.

The inventors of the present invention have intensively studied in order to solve the above problems, and as a result, the inventors have found that a feed containing cashew nut shell liquid suppresses methane production and promotes propionic acid production in a rumen. Thus, the present invention has been accomplished.

The present application discloses:
(1) A rumen fermentation improving agent, comprising cashew nut shell liquid.
(2) A composition for a feed for improving rumen fermentation, comprising the rumen fermentation improving agent according to (1).
(3) A feed for improving rumen fermentation, comprising the composition for a feed for improving rumen fermentation according to (2).
(4) A composition for a feed for improving rumen fermentation, comprising cashew nut shell liquid.
(5) A feed for improving rumen fermentation, comprising cashew nut shell liquid.
(6) A feed for improving rumen fermentation according to (3) or (5), which is for a ruminant.
(7) A method of breeding a ruminant, comprising allowing the ruminant to ingest the feed for improving rumen fermentation according to any one of (3), (5), and (6).
(8) Use of cashew nut shell liquid in manufacturing a rumen fermentation improving agent.
(9) Use of cashew nut shell liquid in manufacturing a composition for a feed for improving rumen fermentation.
(10) Use of cashew nut shell liquid in manufacturing a feed for improving rumen fermentation.
(11) A method of improving rumen fermentation of a domestic animal, comprising administering cashew nut shell liquid to the domestic animal.

By allowing a ruminant to ingest a feed into which the rumen fermentation improving agent or the composition for a feed of the present invention is mixed, or by allowing a ruminant to ingest the feed of the present invention, methane production may be suppressed and propionic acid production may be promoted. The feed of the present invention may be preferably used for breeding ruminants such as cattle, goats, and sheep.

### Best Mode for carrying out the Invention

A rumen fermentation improving agent of the present invention includes cashew nut shell liquid (CNSL). Here, rumen fermentation-improving effects include a methane production-suppressing effect and a propionic acid production-promoting effect in a rumen.

The cashew nut shell liquid to be used in the present invention is an oily liquid contained in the shell of the seed of a cashew nut tree (*Anacardium occidentale L.*). The cashew nut shell liquid contains, as the components thereof, anacardic acids, cardanol, and cardol. In particular, in order to stably exhibit rumen fermentation-improving effects, cashew nut shell liquid in which anacardic acids are contained at a ratio of 40 mass% or more is preferred, and cashew nut shell liquid in which anacardic acids are contained at a ratio of 50 mass% or more is more preferred. There are three kinds of anacardic acids: an anacardic acid having three double bonds at 8-, 11-, and 14-positions (hereinafter, referred to as 15:3); an anacardic acid having two double bonds at 8- and 11-positions (hereinafter, referred to as 15:2); and an anacardic acid having one double bond at 8-position (hereinafter, referred to as 15:1). From the viewpoint of rumen function-improving effects, a ratio of (15:1) : (15:2) : (15:3) is preferably 80 to 100 : 85 to 120 : 140 to 180 and more preferably 90 to 95 : 100 to 110 : 150 to 160.
On the other hand, in the case of using a pure anacardic acid, there arises a problem that the production cost becomes high from the following reasons: the number of steps for producing the pure anacardic acid increases, because an anacardic acid needs to be subjected to solvent extraction from cashew nut shells or cashew nut shell liquid followed by fractionation; an explosion-proof facility is required, because a solvent is employed; and a facility that enables to complete removal of the solvent, because when the used solvent is remained in the anacardic acid, the anacardic acid cannot be used as a feed.
Therefore, the cashew nut shell liquid obtained by squeezing oil from cashew nut shells is preferred from the viewpoint that the production cost can be reduced. Further, a ratio of the above-mentioned anacardic acids, cardanol, and cardol is preferably 50 to 80 : 1 to 30 : 5 to 40 from the viewpoint of the rumen function-improving effects, and is more preferably 55 to 65 : 5 to 20 : 10 to 30.
The anacardic acids, cardanol, and cardol each have an antioxidant action, and thus it is considered that these components suppress the oxidation of each other. Therefore, the rumen fermentation improving agent of the present invention containing cashew nut shell liquid can exhibit rumen fermentation-improving effects more stably than by using anacardic acids, cardanol, or cardol alone, each having double bond(s) to be oxidized easily.

In general, the cashew nut shell liquid can be obtained by heating, but in the present invention, it is preferred that the cashew nut shell liquid be extracted under unheated conditions in order to prevent anacardic acids, which are main constituent components, from being denatured by heat. For example, the cashew nut shell liquid can be obtained by an extracting method involving compressing the shell of a cashew nut or a method described in JP 08-231410 A.
The cashew nut shell liquid used in the present invention may also be a liquid obtained by pulverizing/crushing the shell of a cashew nut.
For the cashew nut shell liquid used in the present invention, a commercially-available product which is not subjected to heat treatment may also be used.
The cashew nut shell liquid used in the present invention is preferably unheated cashew nut shell liquid from the viewpoint of the stability of active ingredients.

The content of the cashew nut shell liquid in the rumen fermentation improving agent of the present invention is, from the viewpoints of effects and costs, preferably 1 mass% to 100 mass%, more preferably 5 mass% to 90 mass%, and still more preferably 10 mass% to 80 mass%, with respect to a total amount of the rumen fermentation improving agent. When the content is 1 mass% or more, the rumen fermentation effect can be exhibited effectively with a certain amount of the rumen fermentation improving agent. Further, a stock solution of cashew nut shell liquid may be directly orally administered.

The rumen fermentation improving agent of the present invention may further contain, in addition to the cashew nut shell liquid, an arbitrary component(s) such as a component which is effective for the growth promotion of a ruminant, a nutritional supplement component, a component for enhancing the preservation stability. Examples of the arbitrary components include the followings: probiotics such as *Enterococcus*, *Bacillus*, and *Bifidus*; enzymes such as amylase and lipase; vitamins such as L-ascorbic acid, choline chloride, inositol, and folate; minerals such as potassium chloride, iron citrate, magnesium oxide, and phosphates; amino acids such as DL-alanine, DL-methionine, L-lysine; organic acids such as fumaric acid, butyric acid, lactic acid, acetic acid, and their salts; antioxidants such as ethoxyquin, dibutylhydroxytoluene, butylhydroxy anisole, ferulic acid, vitamine C, and vitamine E; fungicides such as calcium propionate; binders such as carboxylmethyl cellurose (CMC), casein sodium, and sodium polyacrylate; emulsifiers such as lecithin, glycerin fatty acid ester and sorbitan fatty acid ester; pigments such as astaxanthin and canthaxanthin; and flavoring agents such as various esters, ethers, and ketones.

The formulation of the rumen fermentation improving agent of the present invention is not particularly limited, and the agent may be in an arbitrary form such as powder, liquid, solid, a tablet, a capsule, or emulsion. The rumen fermentation improving agent of the present invention can be produced by mixing cashew nut shell liquid and, if required, an arbitrary component, and forming the mixture into a preparation. Note that, depending on the form of the formulation, the pulverized/crushed product of the above-mentioned cashew nut shell or the cashew nut shell as it is without being subjected to any treatment is mixed with another arbitrary component, and the mixture can be used as the rumen fermentation improving agent of the present invention. In addition, without being mixed with another arbitrary component, the pulverized/crushed product as it is or the cashew nut shell as it is may be used as the rumen fermentation improving agent, and the rumen fermentation improving agent itself may be used as a composition for a feed or a feed.

The composition for a feed of the present invention includes cashew nut shell liquid. Further, the composition for a feed of the present invention may also include the rumen fermentation improving agent. The content of the cashew nut shell liquid in the composition for a feed of the present invention is, from the viewpoints of effects and costs, preferably 0.5 to 500,000 mass ppm, more preferably 5 to 100,000 mass ppm, and still more preferably 50 to 50,000 mass ppm with respect to a dry mass of the composition for a feed. The composition for a feed of the present invention is mixed with another feed component used in pet foods and supplements for pets (hereinafter referred to as feed), to thereby produce a feed. The kind of the feed and the components other than the cashew nut shell liquid are not particularly limited. The feed is preferably for ruminants.

The feed of the present invention includes cashew nut shell liquid. Further, the feed of the present invention may also include the composition for a feed. The content of the cashew nut shell liquid in the feed of the present invention is, from the viewpoints of effects and costs, preferably 0.5 to 50,000 mass ppm, more preferably 5 to 10,000 mass ppm, and still more preferably 50 to 5,000 mass ppm with respect to a dry mass of the feed. It should be noted that, in the rumen juice of an actual ruminant, it is considered that the cashew nut shell liquid is diluted about 10 fold, and hence, it is suggested that the cashew nut shell liquid be administered about 10 times the content of the effective content *in vitro,* in the case of being used *in vivo*.

The feed of the present invention can be produced by adding cashew nut shell liquid or a composition for a feed including the cashew nut shell liquid as it is to a feed component and mixing the resultant. On this occasion, when a powdery or solid composition for a feed is used, the form of the composition for a feed may be modified into a liquid form or a gel form for the purpose of facilitating the mixing process. In this case, the following may be used as a liquid carrier: water; a vegetable oil such as soybean oil, rapeseed oil, or corn oil; or a water-soluble polymer compound such as a liquid animal oil, polyvinylalcohol, polyvinylpyrrolidone, or polyacrylic acid. Further, in order to keep the uniformity of the cashew nut shell liquid in the feed, the feed also preferably contains alginic acid, sodium alginate, xanthan gum, casein sodium, gum arabic, guar gum, or water-soluble polysaccharides such as tamarind seed polysaccharide.

The species of animals that ingest the feed of the present invention is preferably ruminants. The feed of the present invention is suitable for breeding, for example, ruminants such as cows, goats, and sheep. The amount of feed ingested by an animal may be appropriately adjusted depending on the animal's species, body weight, age, sex, health condition, feed component, etc. In this case, the amount of cashew nut shell liquid contained in the feed is preferably 0.005 to 500 g per ruminant per day, more preferably 0.5 to 100 g per ruminant per day, and still more preferably 0.5 to 50 g per ruminant per day.
Any method usually used may be adopted as a method of feeding animals and a method of breeding animals depending on the species of animals.

### Examples

### [Example 1]

### Effects of cashew nut shell liquid (CNSL) on gas production and production of volatile fatty acids in vitro

### (1) Sample

Cashew nut shell liquid (CNSL), which was extracted by compressing cashew nut shells, was obtained from Cashew Trading Co., Ltd. For a culture inoculum, there was used rumen juice (quadruple gauze filtrate) collected from a Holstein cow (fitted with rumen cannula) owned by Experiment Farm, Field Science Center for Northern Biosphere, Hokkaido University. The inoculum was diluted two fold with McDougal's artificial saliva (pH 6.8) and used.

### (2) Culture

The culture was performed in such a manner that the concentration of CNSL in a test culture medium was 500 mg/L. 0.05 g of CNSL was dissolved in 1 ml of ethanol, and 100 µl of the solution was added to a Hungate tube. Ethanol was volatilized by leaving the resultant standing for several hours. Then, as a culture substrate, added therein were 0.15 g of corn starch, 0.025 g of blended feed powder, and 0.025 g of powdered orchard grass hay. 10 ml of the above-mentioned diluted rumen juice were added to the mixture, and the tube was sealed with a butyl rubber cap and a plastic screw cap while blowing nitrogen gas to the headspace, to be subjected to anaerobic culture in a water bath (37°C, 18 hours).
Treatments include an additive-free (ethanol only: control) zone and a CNSL zone, and the culture was performed in quintuplicate in each zone.

### (3) Analysis

Methane, hydrogen, and carbon dioxide were analyzed by a TCD gas chromatography. The concentration and composition of total volatile fatty acids (VFA) were determined by FID gas chromatography.

### (4) Results

### (i) Gas production

Table 1 shows the results of gas production.
Carbon dioxide and methane were decreased significantly by adding CNSL. In particular, methane was hardly detected (98% suppression). The accumulation of hydrogen accompanied by the decrease in methane was not observed, and hydrogen produced in anaerobic fermentation was smoothly transferred to an alternative hydrogen consuming system.

[Table 1]

**Table 1 Gas production in vitro from rumen culture medium added with CNSL**

| | CO₂ (ml) | CH₄ (ml) | H₂ (ml) |
|---|---|---|---|
| Control zone | 2.54 ±0.65ₐ | 0.78 ±0.27ₐ | 0.007 ±0.002 |
| CNSL-added zone | 1.82 ±0.30_{b} | 0.01 ±0.01_{b} | 0.013 ±0.006 |

| | | | |
|---|---|---|---|
| CNSL was added at a final concentration of 500 mg/L. | | | |

Culture was performed in quintuplicate anaerobically at 37°C for 18 hours.

Significant differences are present between different alphabetical letters.

### (ii) Production of volatile fatty acids (VFA)

Table 2 shows the results of the production of VFA.
The VFA concentration was not changed by adding CNSL, and thus there was no suppression of fermentation. However, the fermentation pattern changed remarkably, acetic acid production and butyric acid production decreased significantly, and propionic acid production increased significantly.

[Table 2]

**Table 2 Production of volatile fatty acids in vitro from rumen culture medium added with CNSL**

| | Total VFA (mM/dl) | Acetic acid (%) | Propionic acid (%) | Butyric acid (%) |
|---|---|---|---|---|
| Control zone | 7.61 ±0.50 | 67.62 ±1.78ₐ | 21.59 ±0.78ₐ | 9.68 ±0.22ₐ |
| CNSL-added zone | 7.31 ±0.73 | 54.75 ±3.09_{b} | 40.50 ±3.68_{b} | 4.31 ±0.50_{b} |

| | | | | |
|---|---|---|---|---|
| Significant differences are present between different alphabetical letters. "%" refers to percentage with respect to the total VFA. | | | | |

The results link well with decrease in methane production, and propionic acid production improved remarkably as an alternative consuming system of hydrogen.

### [Example 2]

### Antibacterial action of CNSL

For examining the antibacterial action of CNSL, the following strains were each cultured in a brain-heart-infusion medium (manufactured by NISSUI PHARMACEUTICAL CO., LTD.) at 37°C for a day: *Staphylococcus* aureus strain isolated from a bovine; *Streptococcus bovis* DSM20065 strain; *Bacillus subtilis* NBRC3009 strain; *Escherichia coli* ATCC11303 strain; *Pseudomonas aeruginosa* NBRC12689 strain; and *Saccharomyces cerevisiae* NBRC10217 strain. Into the brain-heart-infusion medium to which CNSL was added, 10 uL each of culture media of the above-mentioned strains were inoculated, and the resultant was cultured at 37°C for two days, to thereby calculate a minimum growth-inhibitory concentration (MIC).
Table 3 shows the results.

**[Table 3]**

| | | MIC (µg/ml) |
|---|---|---|
| Gram-positive bacteria | *Staphylococcus aureus* isolated from a bovine | 6.25 |
| | *Streptococcus bovis* DSM20065 | 9.38 |
| | *Bacillus subtilis* NBRC3009 | 6.25 |
| Gram-negative bacteria | *Escherichia coli* ATCC11303 | >1600 |
| | Pseudomonas *aeruginosa* NBRC12689 | >1600 |
| Fungus | *Saccharomyces cerevisiae* NBRC10217 | >1600 |

CNSL does not have an antibacterial action against Gram-negative bacteria, while CNSL has a high antibacterial action against Gram-positive bacteria. That is, CNSL has a selective antimicrobial action against rumen microorganisms.

### [Example 3]

### Effects of CNSL concentration on production of methane and volatile fatty acids in vitro

### (1) Sample

Cashew nut shell liquid (CNSL), which was extracted by compressing cashew nut shells, was obtained from Cashew Trading Co., Ltd. For a culture inoculum, there was used rumen juice (quadruple gauze filtrate) collected from a Holstein cow (fitted with rumen cannula) owned by Experiment Farm, Field Science Center for Northern Biosphere, Hokkaido University. The inoculum was diluted two fold with McDougal's artificial saliva (pH 6.8) and used.

### (2) Culture

The culture was performed in such a manner that the concentrations of CNSL in test culture media were 50, 100, 250, and 500 mg/L. CNSL was dissolved in 1 ml of ethanol, and 100 ml of the solution was added to a Hungate tube. Ethanol was volatilized by leaving the resultant standing for several hours. Then, as a culture substrate, added therein were 0.15 g of corn starch, 0.025 g of blended feed powder, and 0.025 g of powdered orchard grass hay. 10 ml of the above-mentioned diluted rumen juice were added to the mixture, and the tube was sealed with a butyl rubber cap and a plastic screw cap while blowing nitrogen gas to the headspace, to be subjected to anaerobic culture in a water bath (37°C, 18 hours) .
Treatments include an additive-free (ethanol only: control) zone and a CNSL zone, and the culture was performed in quintuplicate in each zone.

### (3) Results

**[Table 4]**

| Concentration of CNSL (mg/L) | 0 | 50 | 100 | 250 | 500 |
|---|---|---|---|---|---|
| Amount of methane production (ml) | 2.43±0.30ₐ | 1.88±0.22ₐ | 1.64±0.40ₐ | 0.45±0.02_{b} | 0.12±0.04_{c} |
| Total VFA (mmol) | 79.94±2.32ₐ | 77.91±7.59_{abc} | 80.46±3.64ₐ | 67.56±4.39_{bc} | 68.70±1.81_{b} |
| Propionic acid (mmol) | 19.84±0.21ₐ | 20.09±1.39ₐ | 23.43±1.04_{c} | 37.63±2.01_{b} | 38.07±0.85_{b} |

| | | | | | |
|---|---|---|---|---|---|
| Significant differences are present between different alphabetical letters. | | | | | |

With the addition of 250 mg/L or more of CNSL, methane was hardly detected. In the zone added with 250 mg/L or more of CNSL, the concentration of total volatile fatty acids slightly decreased, but the fermentation pattern changed remarkably. With the addition of 100 mg/L or more of CNSL, propionic acid production increased significantly.

### [Example 4]

### Effects with time of CNSL concentration on production of volatile fatty acids in vitro

### (1) Sample

Cashew nut shell liquid (CNSL), which was extracted by compressing cashew nut shells, was obtained from Cashew Trading Co., Ltd. For a culture inoculum, there was used rumen juice (quadruple gauze filtrate) collected from a Holstein cow (fitted with rumen cannula) owned by Experiment Farm, Field Science Center for Northern Biosphere, Hokkaido University.

### (2) Test

Into a 1,000-ml fermentor, 800 ml of a culture medium in which rumen juice and McDougal's artificial saliva (pH 6.8) were mixed at a ratio of 1:1 (mass) was added, and carbon dioxide was blown thereinto to thereby establish an anaerobic condition. The artificial saliva was continuously fed into the fermentor, and waste liquid was collected as a sample. Generated gas was collected in a GasPak. There was a plate having holes placed inside the fermentor, and owing to slow up-and-down movement of the plate, the contents were mixed. Two nylon mesh bags each of which containing a coarse feed (feed) were placed inside the fermentor at all times, and the older bag of the two bags was replaced with another bag once a day. CNSL was added to the feed in such a manner that the concentrations of CNSL in culture media were 0, 50, 100, and 200 mg/L, and the culture was performed in duplicate in each culture media. The culture period was 21 days. The culture was performed under dark conditions at all times.

### (3) Results

Table 5 shows the amount (ml) of collected gas.

**[Table 5]**

| CNSL (mg/L) | Hydrogen | Methane | Carbon dioxide |
|---|---|---|---|
| 0 | 1.30 | 67.07 | 500.84 |
| 50 | 1.76 | 50.34 | 531.11 |
| 100 | 4.99 | 38.04 | 577.25 |
| 200 | 4.36 | 23.91 | 511.18 |

The amount of methane was decreased by adding CNSL.
Table 6 shows the concentration (mmol/dl) of total volatile fatty acids (VFA) in the collected rumen juice.

**[Table 6]**

| | CNSL (mg/L) | | | |
|---|---|---|---|---|
| Day | 0 | 50 | 100 | 200 |
| 0 | 10.77 | 11.43 | 11.37 | 10.96 |
| 3 | 10.80 | 10.89 | 12.13 | 11.40 |
| 6 | 11.73 | 13.03 | 13.39 | 12.69 |
| 9 | 12.56 | 12.76 | 12.99 | 12.83 |
| 12 | 11.70 | 11.89 | 13.80 | 11.70 |
| 15 | 10.49 | 11.79 | 11.77 | 10.86 |
| 18 | 11.11 | 12.17 | 11.83 | 11.57 |
| 21 | 10.65 | 11.35 | 12.19 | 11.21 |

The total VFA concentration was not changed by adding CNSL (i.e. there is no suppression of fermentation).
Table 7 shows the molar ratio (%) of acetic acid in the collected rumen juice, Table 8 shows the molar ratio (%) of propionic acid in the collected rumen juice, and Table 9 shows the molar ratio (%) of butyric acid in the collected rumen juice.

[Table 7]

**Table 7 Molar ratio of acetic acid**

| | CNSL (mg/L) | | | |
|---|---|---|---|---|
| Day | 0 | 50 | 100 | 200 |
| 0 | 48.6 | 95.9 | 44.1 | 41.7 |
| 3 | 48.9 | 45.9 | 44.3 | 41.4 |
| 6 | 49.0 | 46.3 | 44.7 | 41.0 |
| 9 | 50.2 | 46.6 | 45.1 | 41.6 |
| 12 | 48.6 | 46.2 | 45.1 | 41.7 |
| 15 | 50.2 | 45.4 | 45.6 | 40.6 |
| 18 | 48.2 | 45.3 | 43.6 | 41.3 |
| 21 | 48.1 | 45.5 | 43.4 | 40.9 |

[Table 8]

**Table 8 Molar ratio of propionic acid**

| | CNSL (mg/L) | | | |
|---|---|---|---|---|
| Day | 0 | 50 | 100 | 200 |
| 0 | 26.8 | 27. 6 | 30.6 | 35.8 |
| 3 | 26.5 | 27.4 | 30.7 | 35.9 |
| 6 | 26.1 | 27.4 | 30.8 | 36.1 |
| 9 | 25.8 | 27.2 | 31.2 | 36.1 |
| 12 | 26.1 | 27.3 | 31.1 | 35.9 |
| 15 | 25.4 | 27.1 | 30.4 | 36.0 |
| 18 | 26.2 | 27.7 | 31.5 | 34.5 |
| 21 | 25.9 | 27.3 | 31.6 | 36.0 |

[Table 9]

**Table 9 Molar ratio of butyric acid**

| | CNSL (mg/L) | | | |
|---|---|---|---|---|
| Day | 0 | 50 | 100 | 200 |
| 0 | 15.3 | 15.7 | 14.5 | 12.7 |
| 3 | 15.4 | 15.7 | 13.9 | 12.6 |
| 6 | 15.1 | 15.2 | 13.6 | 12.7 |
| 9 | 14.5 | 15.2 | 13.1 | 12.3 |
| 12 | 15.3 | 15.1 | 13.2 | 12.5 |
| 15 | 14.4 | 15.4 | 13.0 | 12.6 |
| 18 | 15.2 | 15.1 | 13.1 | 13.2 |
| 21 | 14.8 | 15.1 | 13.1 | 12.3 |

With the addition of 100 mg/L of CNSL and 200 mg/L of CNSL, the fermentation pattern changed remarkably, acetic acid production and butyric acid production decreased, and propionic acid production increased.

### [Example 5]

### Effects with time of CNSL administration in vivo

### (1) Sample

Four sheep fitted with rumen cannula were each provided with a feed (concentrated feed : hay = 3:7 (volume)) in an amount equivalent to 1.4 mass% of the weight of the each sheep.
A first sampling of rumen contents was performed before starting the administration of CNSL. As for the dose of CNSL, rumen function-improving effects were observed with the addition of 100 mg/L or more of CNSL in the test *in vitro.* In order to allow the concentration of CNSL in the rumen juice of the sheep to be 100 mg/L, it is required to mix 0.14 to 0.28 mass% (1,400 to 2, 800 mass ppm) of CNSL into the feed, because CNSL is diluted in the rumen juice. Accordingly, 0.14 mass% of CNSL was added to the feed for the first two weeks and the sampling of rumen contents was performed once a week, i.e., twice in total. 0.28 mass% of CNSL was added to the feed for the next two weeks and the sampling of rumen contents was performed once a week, i.e., twice in total. For the next two weeks, only a feed in which CNSL is not added was provided to the sheep, and the sampling of rumen contents was performed once a week, i.e., twice in total.

### (2) Results

Table 10 shows the amount of the produced gas (ml/day/tube) when the collected rumen juice was sealed in a test tube and cultured at 37°C for 24 hours.

**[Table 10]**

| | CNSL dose | Hydrogen | Methane | Carbon dioxide |
|---|---|---|---|---|
| Before starting administration | - | 0.03±0.00 | 0.83±0.42 | 3.44±0.79 |
| First week | 0.14 mass% | 0.04±0.02 | 0.69±0.12 | 3.43±0.79 |
| Second week | 0.14 mass% | 0.03±0.01 | 0.46±0.18 | 2.36±0.72* |
| Third week | 0.28 mass% | 0.07±0.07 | 0.22±0.13** | 1.63±0.57** |
| Fourth week | 0.28 mass% | 0.04±0.01 | 0.32±0.23* | 2.65±0.90 |
| Fifth week | Discontinuation of administration | 0.02±0.00** | 0.44±0.20 | 2.76±0.74 |
| Sixth week | Discontinuation of administration | 0.03±0.01* | 0.73±0.80 | 3.30±2.26 |

| | | | | |
|---|---|---|---|---|
| * P<0.10 compared to before starting administration ** P<0.05 compared to before starting administration | | | | |

The amount of methane was decreased significantly by adding CNSL. The accumulation of hydrogen accompanied by the decrease in methane was not observed, and hence, it is considered that hydrogen produced in anaerobic fermentation was smoothly transferred to an alternative hydrogen consuming system.
Table 11 shows the total VFA concentration (mmol/dl), the molar ratio (%) of acetic acid, the molar ratio (%) of propionic acid, the molar ratio (%) of butyric acid in the collected rumen juice.

**[Table 11]**

| | CNSL dose | VFA concentration | Acetic acid % | Propionic acid % | Butyric acid % |
|---|---|---|---|---|---|
| Before starting administration | - | 3.35±0.4 | 60.8±4.0 | 20.7±3.5 | 15.5±2.6 |
| First week | 0.14 mass% | 3.53±1.04 | 55.4±2.9 | 24.0±3.0 | 16.5±2.6 |
| Second week | 0.14 mass% | 3.43±0.92 | 55.7±1.7* | 23.6±5.9 | 11.8±3.5 |
| Third week | 0.28 mass% | 2.85±1.03 | 46.8±2.5** | 31.0±8.6* | 13.4±6.0 |
| Fourth week | 0.28 mass% | 3.05±0.57 | 53.8±1.6** | 30.5±4.8** | 10.0±3.6 |
| Fifth week | Discontinuation of administration | 3.69±0.77 | 59.7±2.4 | 20.5±5.4 | 13.2±3.7 |
| Sixth week | Discontinuation of administration | 4.10±0.82 | 62.4±1.1 | 17.8±3.6 | 13.3±3.4 |

| | | | | | |
|---|---|---|---|---|---|
| * P<0.10 compared to before starting administration ** P<0.05 compared to before starting administration | | | | | |

The total VFA concentration was not changed by adding CNSL in an amount of 0.14 mass% (i.e. there is no suppression of fermentation). Further, with the addition of CNSL in an amount of 0.28 mass%, the fermentation pattern changed remarkably, acetic acid production decreased significantly, and propionic acid production increased significantly.
Table 12 shows the ammonia concentration (mgN/dl) in the collected rumen juice.

**[Table 12]**

| | CNSL dose | Ammonia concentration |
|---|---|---|
| Before starting administration | - | 22.82±5.00 |
| First week | 0.14 mass% | 23.76±3.81 |
| Second week | 0.14 mass% | 21.71±4.61 |
| Third week | 0.28 mass% | 14.11±6.62* |
| Fourth week | 0.28 mass% | 13.01±7.27* |
| Fifth week | Discontinuation of administration | 26.59±6.86 |
| Sixth week | Discontinuation of administration | 28.80±7.26 |

| | | |
|---|---|---|
| * P<0.10 compared to before starting administration ** P<0.05 compared to before starting administration | | |

With the administration of CNSL, the tendency of decrease in ammonia concentration was observed. The results show that proteolysis or deamination is suppressed and feed efficiency is increased.
The results *in vitro* obtained in Examples 1 to 4 correlated well with the results obtained in Example 5, which used sheep. That is, in an actual rumen, with the addition of CNSL, carbon dioxide and methane decreased significantly, and because the accumulation of hydrogen accompanied by the decrease in methane was not observed at that time, it is considered that hydrogen produced in anaerobic fermentation was smoothly transferred to an alternative hydrogen consuming system. Further, the concentration of total volatile fatty acids was not changed by adding CNSL (i.e. there is no suppression of fermentation). However, the fermentation pattern changed remarkably, acetic acid production decreased significantly, and propionic acid production increased significantly.
The results link well with decrease in methane production, and it is considered that propionic acid production developed smoothly as an alternative consuming system of hydrogen. The above facts were exemplified in the actual rumen of the sheep, and hence, it is considered that the cashew nut shell liquid enhances the utilization efficiencies of energy and protein in domestic animals.

### Industrial Applicability

Methane produced by cattle is a loss of feed energy and is also a greenhouse gas, and therefore, it is an urgent issue to reduce methane production from cattle from the viewpoints of zootechnical science and environmentology. By allowing a ruminant to ingest cashew nut shell liquid when breeding the ruminant, methane production can be suppressed. On the other hand, propionic acid has the highest transformation efficiency of feed hexose energy among the volatile fatty acids and is an original substance of sugar to be changed into glucose after absorption, and hence, promotion of propionic acid production leads to saving of other original substances of sugar (e.g., amino acid). Thus, the feed containing cashew nut shell liquid can enhance the utilization efficiencies of energy and protein in domestic animals.

## Claims

1. Non-therapeutic use of cashew nut shell liquid for improving feed efficiency by improving rumen fermentation in a ruminant.

2. Non-therapeutic use of cashew nut shell liquid for promoting the growth of a ruminant and for improving feed efficiency by improving rumen fermentation in the ruminant.

3. Non-therapeutic use according to claim 1 or 2, wherein said cashew nut shell liquid suppresses production of methane and ammonia in the rumen of a ruminant.

4. Non-therapeutic use according to any of claims 1 to 3, wherein the cashew nut shell liquid is provided in the form of a feed.

## Patentansprüche

1. Nicht-therapeutische Verwendung von Cashewnussschalenflüssigkeit zur Verbesserung der Futterverwertung durch Verbesserung der Pansenfermentation in einem Wiederkäuer.

2. Nicht-therapeutische Verwendung von Cashewnussschalenflüssigkeit zur Förderung des Wachstums eines Wiederkäuers und zur Verbesserung der Futterverwertung durch Verbesserung der Pansenfermentation in dem Wiederkäuer.

3. Nicht-therapeutische Verwendung gemäß Anspruch 1 oder 2, wobei die Cashewnussschalenflüssigkeit die Produktion von Methan und Ammoniak im Pansen des Wiederkäuers unterdrückt.

4. Nicht-therapeutische Verwendung gemäß einem der Ansprüche 1 bis 3, wobei die Cashewnussschalenflüssigkeit in Form eines Futters bereitgestellt wird.

## Revendications

1. Utilisation non thérapeutique de baume de cajou pour améliorer l'efficacité alimentaire en améliorant la fermentation dans le rumen chez un ruminant.

2. Utilisation non thérapeutique de baume de cajou pour promouvoir la croissance d'un ruminant et pour améliorer l'efficacité alimentaire en améliorant la fermentation dans le rumen chez le ruminant.

3. Utilisation non thérapeutique selon la revendication 1 ou 2, dans laquelle ledit baume de cajou supprime la production de méthane et d'ammoniac dans le rumen d'un ruminant.

4. Utilisation non thérapeutique selon l'une quelconque des revendications 1 à 3, dans laquelle le baume de cajou est fourni sous la forme d'un aliment.
